# EUROPEAN PATENT APPLICATION

(11) **EP 1 754 481 A2**
(43) Date of publication of application: **21.02.2007**
(21) Application number: 06122805.2
(22) Date of filing: 20.08.2004
(51) Int. Cl.: A61K 31/4164, A61K 31/00, A61P 27/16

(54) **Use of antifungal agents for treating otitis externa**

(30) Priority: 05.02.2004 US 771330; 26.09.2003 US 505754 P; 20.08.2003 US 496409 P
(62) Divisional of application: 04809597.0
(71) Applicant: Fairfield Clinical Trials LLC, Bridgeport, CT 06606 (US)
(72) Inventor: Lane, Edward M, Connecticut, CT 06883 (US)
(74) Representative: Marchant, James Ian

(57) **Abstract**

This invention relates to a method of treating otitis externa, and in particular otitis externa of fungal etiology, using topical medication, including antifungal agents such as, for example, voriconazole, clotrimazole, ravuconazole, posaconazole, econazole, enilaconazole, miconazole, oxiconazole, sulconazole, and tioconazole.

## Description

### BACKGROUND OF THE INVENTION

### 1. Technical Field

This invention relates to the field of medical science, and in particular to treatment of otitis externa, and particularly otitis externa of fungal etiology, with topical or orally administered antifungal agents, preferably including voriconazole and/or clotrimazole.

### 2. Description of the Background Art

Otitis externa is an inflammation of the external auditory canal which can affect people of all ages. This condition is responsible for considerable pain and morbidity. The cause may be bacterial (usually *Staphylococcus* spp.), viral (for example herpes zoster oticus), traumatic (usually caused by aggressive ear cleaning), collection (or appearance) of moisture or water under a cerumen impaction and/or fungal. Otitis externa infections often involve a mixed population of bacteria and fungus. Fungal otitis externa (otomycosis) is a fungal infection of the external auditory canal and generally is caused by (1) *Aspergillus niger* (80-90% of all cases), (2) *Candida albicans* and other *Candida* spp., (3) *Actinomyces* and (4) *Trichophyton.* Factors such as hot, humid environments, chronic bacterial otitis externa, prior treatment of bacterial otitis externa with topical aminoglycosides or other antibacteriologics and suppressed immunity can predispose patients to fungal otitis externa. The number of persons at risk for this infection is increasing due to the liberal and inappropriate use of systemic antibiotics, the increase in patients undergoing bone marrow transplant, solid organ transplant, aggressive chemotherapy for cancer and patients infected with HIV.

Symptoms of otitis externa can include significant ear canal pruritis, pain (particularly with motion of the external ear), otorrhea (usually foul and purulent), conductive hearing loss and cervical lymphadenitis. Whitish-grey, yellow or black ear canal exudate, erythema and swelling of the canal walls, external auditory canal meatus and tympanic membrane, and a distinctive odor are hallmarks of the condition. Other symptoms may include hearing loss, tinnitus, fever and others. If the infection is severe, it may spread through the skin layers to cartilage and/or bone, and can spread to the face or neck. Necrotizing or malignant otitis externa, a *Pseudomonas* spp. ostiitis of the temporal bone, may occur, especially in adults with diabetes mellitus, both Types I and II, as well as in patients who are immunocompromised. Diagnosis of otitis externa often is confirmed by staining a sample of the exudate with potassium hydroxide (10% KOH) or fungal culture, although most patients are diagnosed empirically.

Treatment of otitis externa involving fungal organisms generally entails vigorous ear canal cleaning (ear toilet), irrigation and acidification. Occasionally, surgical debridement of the ear canal is indicated. Current therapy for fungal otitis externa relies on the use of acidifying solutions (for example acetic acid, with or without hydrocortisone) or topical agents designed for treatment of Athlete's Foot (for example clotrimazole (Lotrimin®). Such topical agents are designed for treatment of candidiasis, but generally are not efficacious for many of the organisms known to cause fungal otitis externa and so have proved ineffective. Topical antibiotic preparations have been in use for many years to treat otitis of bacterial origin. There are, however, no topical or systemic medications indicated for treatment or prophylaxis of fungal otitis externa commercially available at this time.

Orally active antifungal drugs have been described. See United States Patent No. 4,404,216. These drugs have been used effectively for invasive fungal infections due to *Candida, Aspergillis,* and other fungi. Azole antifungal agents such as fluconazole and voriconazole exert their effect by inhibiting cytochrome p450 14a-desmethylase (P45014DM), an enzyme in the steroid biosynthesis pathway. Voriconazole has in vitro antifungal activity against a number of species and is considered to be effective in vivo against *Candida* spp. and *Cryptococcus neoformans* as well as *Aspergillis* spp., including fluconazole-resistant *Candida* species such as *C*. *krusei* and *C*. *guilliermondii.* Fluconazole (Diflucan®), itraconazole (Sporanox®), voriconazole (Vfend®) and clotrimazole (Mycelex®) have been approved by the FDA for various types of invasive fungal infections. These drugs are synthetic triazole antifungal agents, available as tablets for oral administration. Prescribing information for these drugs list the following indications for usage. Fluconazole: vaginal candidiasis; oropharyngeal and esophageal candidiasis; *Candida* urinary tract infections, peritonitis, and systemic *Candida* infections including candidemia, disseminated candidiasis, and pneumoma; and cyptococcal meningitis. Voriconazole: invasive aspergillosis and serious fungal infections caused by *Scedosporium apiospermum* and *Fusarium* spp. Itraconazole: blastomycosis, histoplasmosis and aspergillosis in immunocompromised patients and onychomycosis in non-immunocompromised patients. Fluconazole also has been used to decrease the incidence of candidiasis in patients undergoing bone marrow transplantation who receive cytotoxic chemotherapy and/or radiation therapy.

### SUMMARY OF THE INVENTION

In one aspect, the invention relates to a composition for the topical treatment of otitis externa, which comprises an antifungal agent selected from the group consisting of voriconazole, clotrimazole, ravuconazole, posaconazole, miconazole, oxiconazole, sulconazole, tioconazole, econazole and enilaconazole; and at least one pharmaceutically acceptable excipient.

In a second aspect, the invention relates to the use of a composition as described above for the manufacture of a medicament for the treatment of otitis externa by topical administration. Preferred antifungal agents are voriconazole and clotrimazole.

The composition described above may further comprise an antifungal agent selected from the group consisting of fluconazole (Diflucan®), itraconazole (Sporonox®), clotrimazole, amphotericin B, caspofungin (Cancidas®), micafungin (Mycamine®), terbinafine, naftifine, natamycin, butenafine, amorolfine, ravuconazole, posaconazole, flucytosine, econazole, enilaconazole, miconazole, oxiconazole, saperconazole, sulconazole, terconazole, tioconazole, nikkomycin Z, anidulafungin (LY303366), nystatin, pimaricin, griseofulvin, ciclopirox, haloprogin, tolnaftate, and undecylenate.

For topical treatment, the agent is suitably administered in an amount of about 1 mg/day to about 5,000 mg/day, preferably about 5 mg/day to about 500 mg/day and most preferably about 10 mg/day to about 100 mg/day. Treatment preferably should be administered for one day or at least 3 days, preferably for about 7 days to about 14 days. Treatment can be for 180 days or longer. The medicaments are suitable for treating otitis externa that is non-invasive or invasive.

A number of preferred aspects of the invention will be described below.

### DETAILED DESCRIPTION OF THE INVENTION

Antifungal agents preferably are delivered to the affected tissue in a solution or suspension, by medicine dropper. Such solutions or suspensions generally contain about 1 mg to about 5000 mg antifungal agent per mL of solution or suspension, but may contain about 5 mg to about 2,500 mg agent per mL solution or suspension, and preferably about 10 mg to about 1,000 mg agent per mL solution or suspension. The solution or suspension can be delivered to the ear canal in amounts of about 0.01 mL to about 5 mL, preferably about 0.1 mL to about 1 mL, or any amount sufficient to fill the canal volume. An ear wick may be used to assist penetration of the agent into the ear canal according to methods known in the art.

Typical treatments with topical formulations involve administration of 200 mg voriconazole twice daily for 10 days. The length of treatment preferably is at least 10 days but may extend from 1 day to about 14 days, or until the symptoms are resolved. Preferably, treatment continues for 5 days after resolution of symptoms to lessen chance of recurrence.

Solutions and suspensions of these types are known in the art and may contain any conventional or pharmaceutically acceptable and suitable excipients. Alternatively, the azole or other antifungal agents may be formulated as an ointment, lotion, cream, tincture, paste, aqueous or anhydrous gel, or powder according to traditional methods known in the pharmaceutical arts and using any conventional and acceptable pharmaceutical excipient or excipients that are known in the art. Topical preparations according to the invention generally are formulated as a liquid and are applied as ear drops, for example using about 4 drops, to the affected ear canal with eardrum held independently. Other methods for administration of other types of topical formulations are known in the art.

Formulations of azole antifungal agents suitable for use with this invention may contain additional active ingredients in addition to inert pharmaceutical excipients. For example, topical formulations may include hydrocortisone or other corticosteroid agents to assist in reducing inflammation. Such corticosteroids (for example hydrocortisone or dexamethasone) are able to provide synergistically improved effects in topical formulations. Formulations may contain anesthetic agents such as lidocaine or pontocaine or antibacterial agents, if desired.

Formulations according to the invention preferably contain voriconazole, which is effective against *Aspergillis* spp., a common cause of fungal otitis externa. Other agents which may form part of the invention in addition to the agents mentioned in claim 1 include itraconazole (Sporonox®), fluconazole (Diflucan®), ketoconazole, enilaconazole, econazole, saperconazole, oxiconazole, clotrimazole, amphotericin B, caspofungin (Cancidas®), micafungin (Mycamine®), terbinafine, naftifine, natamycin, butenafine, amorolfine, ravuconazole, posaconazole, flucytosine, miconazole, sulconazole, terconazole, tioconazole, nikkomycin Z, anidulafungin (LY303366), nystatin, pimaricin, griseofulvin, ciclopirox, haloprogin, tolnaftate, and undecylenate.

Compositions of the invention may contain an antifungal agent which is effective for the particular causative species of fungus. When the specific causative fungus is not or cannot be identified, or when more than one fungus is present or suspected, voriconazole preferably is used, alone or in combination with another agent.

Topical medications such as powders and creams which are designed to treat athlete's foot sometimes have been used in the ear to treat otitis of fungal origin, however these products, containing clotrimazole or fluconazole for example, do not effectively treat most otitis externa. These agents designed to treat athlete's foot may be effective against some *Candida* species, but are not suitable alone in a general formulation for otitis externa because generally, the causative agent(s) are not known and are not usually *Candida* species. Therefore, these pharmaceutical compositions, which are not effective against *Aspergillus niger,* the most common causative organism, preferably are not used alone but may be used as an additional active ingredient in the inventive compositions.

Preferred topical preparations contain one or more additional antifungal compounds such as those listed above and most preferably contain voriconazole. The primary active ingredient, for example voriconazole, may be combined with a second antifungal agent, an antibacterial agent, an anesthetic, an acidifying agent or buffer, a penetration enhancing agent, an antiinflammatory agent, etc. in a formulation suitable for topical application to the site of infection. Such compositions are effective in the treatment of otitis externa, filling a need in the market, since no effective product is available commercially at this time.

## Claims

1. A composition for the topical treatment of otitis externa, which comprises an antifungal agent selected from the group consisting of voriconazole, clotrimazole, ravuconazole, posaconazole, miconazole, oxiconazole, sulconazole, tioconazole, econazole and enilaconazole; and at least one pharmaceutically acceptable excipient.

2. A composition of claim 1 wherein said antifungal agent is voriconazole.

3. A composition of claim 1 or 2 which further comprises
(a) an antifungal agent selected from the group consisting of fluconazole, itraconazole, clotrimazole, ravuconazole, posaconazole, miconazole, oxiconazole, saperconazole, sulconazole, terconazole, tioconazole, econazole, enilaconazole, amphotericin B, natamycin, nikkomycin Z, caspofungin, micafungin, anidulafungin, terbinafine, naftifine, butenafine, amorolfine, flucytosine, nystatin, pimaricin, griseofulvin, ciclopirox, haloprogin, tolnaftate, and undecylenate.

4. A composition of any of claims 1 to 3 which further comprises an antiinflammatory agent.

5. A composition of any of claims 1 to 4 which further comprises an anesthetic agent.

6. A composition of any of claims 1 to 5 which further comprises a antibacterial agent.

7. A composition of any of claims 1 to 6 which is formulated as an ear drop.

8. Use of a composition according to any of claims 1 to 7 for the manufacture of a medicament for the treatment of otitis externa by topical administration.

9. A use of claim 8 wherein said antifungal agent is administered in an amount of about 1 to about 5,000 mg/day.

10. A use of claim 8 wherein said antifungal agent is administered in an amount of about 5 to about 500 mg/day.

11. A use of claim 8 wherein said antifungal agent is administered in an amount of about 10 mg/day to about 100 mg/day.

12. A use of claim 8 wherein said antifungal agent is administered for at least 3 days.

13. A use of claim 8 wherein said antifungal agent is administered for about 7 days to about 14 days.

14. A use of claim 8 wherein said antifungal agent is administered for up to 180 days.

15. Use of a composition which comprises voriconazole, a corticosteroid, and at least one pharmaceutical excipient suitable for topical administration, for the manufacture of a medicament for the treatment of otitis externa by topical administration.
